**Europäisches Patentamt**

**European Patent Office**

**Office européen des brevets**

(19)

(11) Veröffentlichungsnummer: **0 031 905 B1**

(12)

# EUROPÄISCHE PATENTSCHRIFT

(45) Veröffentlichungstag der Patentschrift:
02.11.83

(21) Anmeldenummer: **80107591.2**

(22) Anmeldetag: **04.12.80**

(51) Int. Cl.³: **C 25 B 3/02**, C 07 D 251/36

(54) **Verfahren zur Herstellung von Mono-(trichlor)-tetra-(monokalium-dichlor)-penta-isocyanurat.**

(30) Priorität: **07.01.80 DE 3000365**

(43) Veröffentlichungstag der Anmeldung:
**15.07.81 Patentblatt 81/28**

(45) Bekanntmachung des Hinweises auf die Patenterteilung:
**02.11.83 Patentblatt 83/44**

(84) Benannte Vertragsstaaten:
**AT BE CH DE FR GB IT LI NL SE**

(56) Entgegenhaltungen:
**DE - A - 2 826 303**
**US - A - 3 835 134**
**US - A - 3 835 135**
**US - A - 3 835 136**

(73) Patentinhaber: **Degussa Aktiengesellschaft,**
**Weissfrauenstrasse 9, D-6000 Frankfurt am Main 1 (DE)**

(72) Erfinder: **Berger, Michael, Dr., Jennerstrasse 39,**
**D-5303 Hemmerich (DE)**
Erfinder: **Koberstein, Edgar, Dr., Wolfskernstrasse 8,**
**D-8755 Aizenau (DE)**

## Verfahren zur Herstellung von Mono-(trichlor)-tetra-(monokalium-dichlor)-penta-isocyanurat

Es ist bekannt, Mono-(trichlor)-tetra-(monokalium-dichlor)-penta-isocyanurat herzustellen, indem in wässrigem Medium bei Temperaturen von 5 bis 50°C entweder Trikaliumcyanurat mit Chlor oder Kaliumdichlorisocyanurat mit Chlorwasserstoff oder mit Trichlorisocyanursäure umgesetzt wird (US-PS 3 150 132). Es ist ausserdem bekannt, das Mono-(trichlor)-tetra-(monokalium-dichlor)-penta-isocyanurat entsteht, wenn Kaliumdichlorisocyanurat-Monohydrat und Trichlorisocyanursäure als Feststoffe unter hohem Druck zusammengebracht werden (US-PS 4 118 569). Ausgangssubstanz ist in jedem Fall Cyanursäure oder letztlich Cyanurchlorid. Es müssen zunächst die für die Umsetzungen vorgesehenen Substanzen in zum Teil aufwendiger Weise aus der Cyanursäure oder dem Cyanurchlorid hergestellt werden. Die Ausbeuten an Mono-(trichlor)-tetra-(monokalium-dichlor)-penta-isocyanurat, bezogen auf die Cyanursäure oder das Cyanurchlorid als Ausgangssubstanz, liegen unter 80% und sind daher unbefriedigend.

Es wurde nun ein elektrolytisches Verfahren zur Herstellung von Mono-(trichlor)-tetra-(monokalium-dichlor)-penta-isocyanurat gefunden, das dadurch gekennzeichnet ist, dass Cyanurchlorid in einem wässrigen, Kaliumionen enthaltendem Medium an einer Anode umgesetzt wird. Bei diesem Verfahren wird auf einfache Weise das Cyanurchlorid unmittelbar in das Mono-(trichlor)-tetra-(monokalium-dichlor)-penta-isocyanurat übergeführt und es wird hierbei eine wesentlich bessere Ausbeute als bei den bekannten Verfahren erzielt.

Erfindungsgemäss erfolgt die Umsetzung des Cyanurchlorids in wässrigem Medium. Die Konzentration des Cyanurchlorids kann weitgehend beliebig gewählt werden. Im allgemeinen ist es vorteilhaft, wenn je g Cyanurchlorid 2 bis 50 ml, insbesondere 5 bis 20 ml, Wasser vorliegen. Der pH-Wert des Mediums wird während der Umsetzung zweckmässigerweise auf 1 bis 12, vorzugsweise auf 3 bis 7, insbesondere auf 3 bis 6, gehalten. Zur Einstellung des pH-Wertes werden alkalisch wirkende Substanzen zugesetzt. Vorzugsweise wird Kaliumhydroxid verwendet. In manchen Fällen kann es vorteilhaft sein, zusätzlich ein Kaliumsalz, vorzugsweise Kaliumchlorid, zuzugeben. Das Verhältnis Kaliumionen zu Cyanurchlorid kann in weiten Bereichen sowohl stöchiometrisch als auch unter- oder überstöchiometrisch gewählt werden. Im allgemeinen ist es jedoch zweckmässig, dass mindestens stöchiometrische Mengen Kaliumionen vorliegen. Vorzugsweise ist das Molverhältnis Kalium zu Cyanurchlorid 0,8 bis 10, insbesondere 2 bis 7.

Das Medium wird zweckmässigerweise während der Umsetzung auf Temperaturen von 5 bis 50°C, vorzugsweise von 5 bis 30°C, insbesondere von 10 bis 25°C, gehalten.

Erfindungsgemäss erfolgt die Umsetzung des Cyanurchlorids in dem Medium unter der Einwirkung einer Gleichspannung an der Anode. Mit Vorteil wird die Spannung zwischen Anode und Kathode auf 1 bis 20, insbesondere auf 4 bis 8, Volt eingestellt. Die Stromdichte beträgt zweckmässigerweise 100 bis 20 000, vorzugsweise 500 bis 10 000, insbesondere 1000 bis 5000, Ampere je m² Elektrodenfläche.

Für die Umsetzung können alle üblichen Elektroden verwendet werden, die sich zur Einhaltung der Umsetzungsbedingungen eignen und aus Materialien bestehen, die sich gegenüber den umzusetzenden und den bei der Umsetzung entstehenden Substanzen zumindest weitgehend inert verhalten. Geeignet sind vornehmlich Elektroden, die im allgemeinen für die Herstellung von Chlor und Alkali durch Elektrolyse eingesetzt werden. In Frage kommen beispielsweise Graphit-Elektroden oder Metall-Elektroden aus den Platinmetallen oder aus gegebenenfalls mit Platinmetallen plattiertem Eisen, Nickel, Edelstahl oder Titan, als Anoden vorzugsweise Elektroden nach der DE-OS 1 671 422 oder der DE-OS 1 814 567, die aus einem Metallkern und einer metallischen oder metalloxidischen Beschichtung bestehen, insbesondere mit Rutheniumoxid beschichtete Titan-Elektroden.

Bei einer besonders bevorzugten Ausführungsform wird der Kathodenraum von dem Anodenraum getrennt, beispielsweise durch eine Ionenaustauschermembran. In diesem Fall wird der Kathodenraum mit einer verdünnten Lösung von Kaliumhydroxid, die vorzugsweise 1 bis 10, insbesondere 2 bis 4, Gewichtsprozent Kaliumhydroxid enthält, und der Anodenraum mit der als Umsetzungsmedium vorgesehenen Flüssigkeit beschickt, die an Cyanurchlorid weitgehend gesättigt ist, einen pH-Wert von 3 bis 7 aufweist und überstöchiometrische Mengen Kaliumionen enthält. Mit besonderem Vorteil wird diese Verfahrensweise kontinuierlich gestaltet und es werden hierzu die Flüssigkeiten durch den Kathodenraum wie auch durch den Anodenraum in stetigem Strom geführt.

*Beispiel 1*

Es wurde eine Zelle verwendet, in der durch eine Kationenaustauschermembran der Anodenraum vom Kathodenraum getrennt war. Die Kathode war aus Edelstahl (V4A-Stahl) gefertigt und hatte eine Oberfläche von 54 cm². Die Anode bestand aus Titan, das mit Rutheniumoxid beschichtet war, und hatte eine Oberfläche von 30 cm². Zu Beginn wurde der Kathodenraum mit 500 ml einer 3prozentigen wässrigen Kaliumhydroxid-Lösung und der Anodenraum mit einer mittels Kaliumhydroxid auf den pH-Wert 4,5 eingestellten Lösung von 50 g Cyanurchlorid und 75 g Kaliumchlorid in 750 ml Wasser gefüllt. Es wurde bei einer Spannung von 6 Volt und einer Stromstärke von 8 Ampere gearbeitet. Die Temperatur betrug 15°C. Während der Umsetzung wurde in stetigem Strom in den Kathodenraum Wasser eingespeist und in den Anodenraum eine mittels Kaliumhydroxid auf den pH-Wert 4,5 eingestellte 6prozentige wässrige Cyanurchlorid-Lösung, sowie Kaliumhydroxid-Lösung in dem Masse, dass im Anodenraum der pH-Wert stets auf 4,5 gehalten wurde. Aus dem Kathodenraum wurde die dort gebildete Kaliumhydroxid-Lösung laufend abgezogen. Sie wurde zur Einstellung des pH-Wertes in der Cyanurchlorid-Lösung oder im Anodenraum verwendet. Im Anodenraum schied sich das Mono-(trichlor)-tetra-(monokalium-dichlor)-penta-isocyanurat als farblose kristalline Substanz ab. Diese wurde mit der Flüssigkeit, die aus dem Anodenraum ablief, ausgetragen und von der Flüssigkeit durch Filtration getrennt. Die Flüssigkeit wurde durch Zugabe von Cyanurchlorid auf 6% Gehalt gebracht, mittels Kaliumhydroxid auf den pH-Wert 4,5 eingestellt und im Kreislauf in den Anodenraum zurückgeführt. Das abfiltrierte Mono-(trichlor)-tetra-(monokalium-dichlor)-penta-isocyanurat wurde mit wenig Wasser gewaschen und getrocknet. Es hatte einen Gehalt an aktivem Chlor von 67%. Die Ausbeute betrug stündlich 14,1 g, entsprechend 98%, bezogen auf das eingesetzte Cyanurchlorid.

*Beispiel 2*

Es wurde wie nach Beispiel 1 verfahren, jedoch wurde die Temperatur des Mediums in der Zelle auf 25°C gehalten. Die Ausbeute betrug stündlich 13,8 g, entsprechend 95%, bezogen auf das eingesetzte Cyanurchlorid.

*Beispiel 3*

Es wurde wie nach Beispiel 1 verfahren, jedoch wurde bei einer Stromstärke von 6 Ampere gearbeitet. Die Ausbeute betrug stündlich 14,0 g, entsprechend 97%, bezogen auf das eingesetzte Cyanurchlorid.

*Beispiel 4*

Es wurde wie nach Beispiel 1 verfahren, jedoch wurde eine Anode aus Platin (Oberfläche 30 cm$^2$) verwendet und bei einer Stromstärke von 10 Ampere gearbeitet. Die Ausbeute betrug stündlich 13,5 g, entsprechend 94%, bezogen auf das eingesetzte Cyanurchlorid.

**Patentansprüche**

1. Elektrolytisches Verfahren zur Herstellung von Mono-(trichlor)-tetra-(monokalium-dichlor)-penta-isocyanurat, dadurch gekennzeichnet, dass Cyanurchlorid in einem wässrigen, Kaliumionen enthaltenden Medium an einer Anode umgesetzt wird.

2. Verfahren nach Anspruch 1, dadurch gekennzeichnet, dass das Umsetzungsmedium einen pH-Wert von 3 bis 7 aufweist.

3. Verfahren nach Anspruch 1 oder 2, dadurch gekennzeichnet, dass die Umsetzung bei Temperaturen von 5 bis 30°C durchgeführt wird.

4. Verfahren nach einem oder mehreren der Ansprüche 1 bis 3, dadurch gekennzeichnet, dass bei einer Spannung von 4 bis 8 Volt und einer Stromdichte von 1000 bis 5000 Ampere je m$^2$ Elektrodenfläche gearbeitet wird.

5. Verfahren nach einem oder mehreren der Ansprüche 1 bis 4, dadurch gekennzeichnet, dass die Umsetzung in einem vom Kathodenraum getrennten Anodenraum vorgenommen wird.

**Claims**

1. An electrolytic process for the production of mono-(trichloro)-tetra-(monopotassium-dichloro)-penta-isocyanurate, characterised in that cyanuric chloride is reacted in an aqueous, potassium ion-containing medium at an anode.

2. A process according to claim 1, characterised in that the reaction medium has a pH of from 3 to 7.

3. A process according to claim 1 or 2, characterised in that the reaction is carried out at a temperature of from 5 to 30°C.

4. A process according to one or more of claims 1 to 3, characterised in that it is carried out under a voltage of from 4 to 8 volts and with a current density of from 1000 to 5000 amps per square meter of electrode surface.

5. A process according to one or more of claims 1 to 4, characterised in that the reaction is carried out in an anode chamber which is separated from the cathode chamber.

**Revendications**

1. Procédé électrolytique d'obtention du mono-(trichloro)-tétra-(monopotassium-dichloro)-penta-isocyanurate, procédé caractérisé en ce que l'on fait réagir du chlorure de cyanuryle, dans un milieu aqueux, contenant des ions potassium, sur une anode.

2. Procédé selon la revendication 1, caractérisé en ce que le milieu réactionnel a un pH de 3 à 7.

3. Procédé selon l'une des revendications 1 ou 2, caractérisé en ce que la réaction est effectuée à une température de 5 à 30°C.

4. Procédé selon l'une quelconque des revendications 1 à 3, caractérisé en ce que l'on opère sous une tension de 4 à 8 volts et avec une densité de courant de 1000 à 5000 ampères par m$^2$ de surface d'électrode.

5. Procédé selon l'une quelconque des revendications 1 à 4, caractérisé en ce que la réaction s'effectue dans un compartiment anodique séparé du compartiment cathodique.